# EUROPEAN PATENT SPECIFICATION

(11) **EP 1 867 355 B1**
(45) Date of publication and mention of the grant of the patent: **31.08.2016**
(21) Application number: 07110040.8
(22) Date of filing: 12.06.2007
(51) Int. Cl.: A61M 1/36, A61M 1/02, A61L 2/00

(54) **A system for photoradiation of blood or fractions thereof and relative procedure**
System zur Photobestrahlung von Blut oder Bruchstücken davon und entsprechendes Verfahren
Système pour la photoradiation de sang ou de fractions de sang et procédé correspondant

(30) Priority: 15.06.2006 GB 0611906
(43) Date of publication of application: 19.12.2007
(73) Proprietor: PELHAM CRESCENT S.r.l., 87100 Cosenza (IT)
(72) Inventor: Dinu, Florian, Vaslui (RO)
(74) Representative: Zoli, Filippo

(56) References cited:
- EP-A- 1 025 872
- EP-A1- 1 512 419
- EP-A1- 1 576 973
- WO-A-2004/033081
- US-A1- 2003 072 676

## Description

The present invention relates to a system for the photoradiation of blood or fractions thereof.

Extracorporeal photoimmunotherapy is a therapy capable of modifying the immunity reactions of an organism (for which reason it is called immunomodulating therapy) and the first phase of the therapy is constituted of a separation of the leukocytes from the blood by means of centrifugal action (known in medical terminology as leukoapheresis); the separation is intended to collect a cellular concentrate of lymphocytes and mononuclear cells.

The second phase of the photoimmunotherapy is constituted of the addition of a photoactivatable drug to the cells collected and the exposure of everything to a either UVA or UVB-type ultraviolet light; the photoactivatable drug utilised is constituted of psoralene and the currently preferred and utilised drug is 8-metoxipsoralene (8-MOP).

At the end of the aforesaid two phases of which the immunotherapy is composed, the preparation is reinfused into the patient and therefore it seems evident that in this case the photoimmunotherapy is of an extracorporeal and autologous type.

It seems interesting to note that the use of certain plants containing high quantities of psoralene together with the subsequent exposure to solar light of patients suffering from cutaneous alterations such as vitiligo and psoriasis has been known since 1400 B.C. in India and there are indications concerning it from ancient Egypt and likewise its effectiveness is known.

Extracorporeal photoimmunotherapy was born in the 1980s and likewise, equipment suitable for the photoradiation of blood was conceived for the execution of photopheresis or extracorporeal photoimmunotherapy and the descriptions abound in medical literature. Originally, the procedure began with the radiation of leukocytes taken from patients suffering from T cell cutaneous lymphoma with UVA rays following administration of a psoralene by mouth. Later on, in the 1990s then, the use of 8-MOP was introduced, which was added directly to the bag of leukocytes taken from the centrifugation before the bag underwent treatment with UVA rays.

At present, the practice of extracorporeal photoimmunotherapy is utilised in the treatment of numerous important pathologies in order to promote and stimulate an immune response: apart from the case of T cell cutaneous lymphoma, also in the event of rejection following a bone marrow transplant, the transplant of solid organs such as hearts, lungs, livers and kidneys and in autoimmune-based illnesses such as progressive systematic sclerosis, rheumatoid arthritis, psoriatic arthritis and systematic erythematosus lupus.

As far as known documents dealing with photoimmunotherapy of blood and its derivatives are concerned, the following documents should be noted: US 4,573,962, US 6,491,656 and EP 1 576 973.

The teaching of document US 4,573,962 concerns a device suitable for radiation of blood in order to photoactivate a reagent substance mixed with the blood, the device being also suitable for reinfusing the radiated blood into the patient.

Document US 6,491,656 describes and claims a device suitable for controlling the movements of fluids over the course of an extracorporeal blood treatment. This device is comprises a case featuring a cavity in which both a plurality of inlets and outlets converge, the inlets being suitable for bringing the fluid to treat into the case, the outlets being used for the treated fluid. Located inside the case, there is at least one valve and a filtering element.

Document EP 1 576 973 deals with an appliance suitable for the radiation of blood and featuring the possibility to allow external radiation dosing thanks to the intervention of a processor; in the device a bag is used which is fitted with meandrous-shaped channels whose feeder pipe is located, in the outlet section, above a pump and whose deviation pipe is positioned in correspondence with the inlet of a recirculation bag.

All the prior documents do not permit certification of the treatment to be executed according to European standards on blood treatment in an extracorporeal environment.

Furthermore, prior art documents US 4,573,962 and US 6,491,656 envisage the separation of the blood with the collection of cellular suspension (so-called "buffy coat") treated directly by an integrated radiation system.

EP 04007887 presents the drawback, among others, that it is lacking both an electronic device capable of receiving, from the appliance, the signals required to manage the various peripheral devices and sensors fitted in the device and also a haematocrit reader. A further limitation of the device in document EP, 1 576 973 is constituted of the fact that, despite the presence of the processor for controlling the appliance's functions, the direct intervention of the operator is required.

EP 1 512 419 discloses an apparatus for photoimmunotherapy of blood or fractions thereof comprising a centrifuge bowl, a couple of bags suitable to contain fractions of blood, an irradiation chamber and a cassette for valuing, pumping and controlling the movement of blood fluids druring a photophere us treatment session.

Conventional devices feature a certain complexity which, on the one hand, determines a burdening of appliance costs, and on the other, does not permit an easy and rapid cleaning and sanitisation of the interior of the appliance should the fluid undergoing treatment leak. Furthermore, the fluid in question may contain particularly infecting agents.

Other drawbacks presented by devices known in the art are constituted of both the lack of devices suitable for anticipating eventual causes of breakage of a disposable assembly utilised in the device and inside which the biological liquid to undergo treatment flows, with the consequent possibility of infecting the operators, and also the impossibility of obtaining the certification of the completed treatment required by law.

A first aim of the present invention is to provide an appliance suitable for extracorporeal photoimmunotherapy which is simpler, less costly and more flexible with respect to the commonly known appliances.

A second aim of the present invention is to render the extracorporeal photoimmunotherapy procedure safer, more standardised and faster.

A still further aim of the present invention is to permit certification of the treatment executed as required by European standards on extracorporeal blood treatment.

In particular, the present invention provides a system for photoimmunotherapy of biological fluid, constituted of blood or fraction thereof, according to claim 1.

This and other characteristics will better emerge from the detailed description that follows of a preferred embodiment, provided in the form of a non-limiting example, with reference to the accompanying drawings, in which:
figure 1 shows a disposable assembly fitted with an incubation bag; and
figure 2 shows the contents of figure 1 but without the incubation bag.

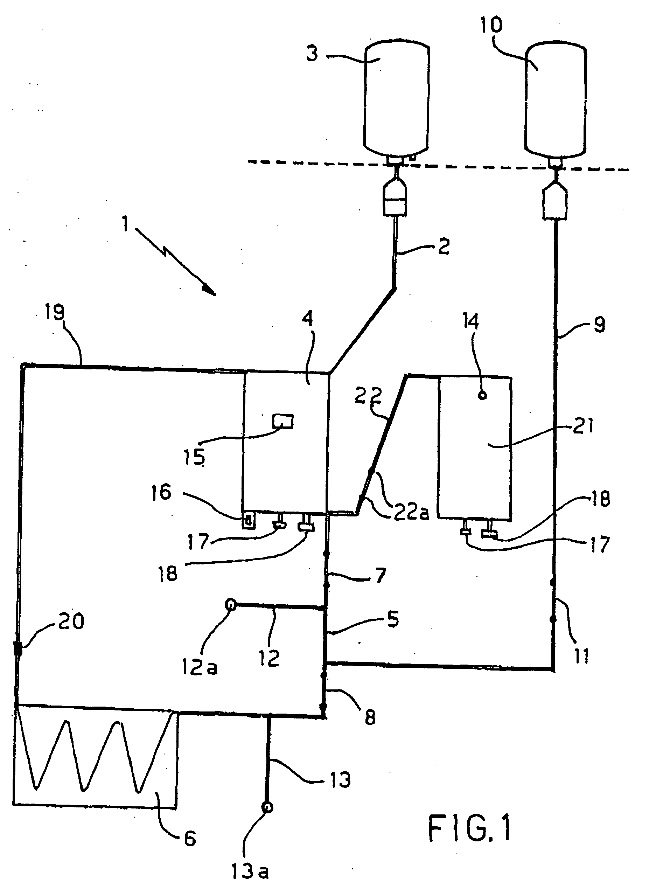

With reference to figure 1, a system embodying the present invention comprises an instrument (not shown) and a disposable assembly 1. There is a viewer situated on the aforesaid instrument suitable for displaying the entire system status, the data entry, the continuous monitoring of the photoradiation process and the system alarms and errors.

There is also a keyboard situated on the instrument by means of which it is possible to enter the data necessary to start, modify and even interrupt the treatment.

Also on the instrument there are seats obtained for positioning the sensors controlled by the devices inserted in the disposable assembly. Inside the instrument there is a photoradiation chamber obtained fitted with two pluralities of UVA light bulbs, one fixed above and the other fixed below the photoradiation chamber, which is also fitted, both above and below, with a plate made of a material which is transparent to UVA rays, for example an acrylic material.

The photoradiation chamber is fitted with two ventilators whose flow of air taken into and emitted from the chamber moves above and below the respective UVA-transparent plate for cooling the radiated cellular suspension. The aforesaid photoradiation chamber is also fitted with a temperature sensor, the sensor being connected to a central processing unit (CPU) and the viewer.

The photoradiation chamber is also fitted with a photometer, i.e. a UVA ray sensor suitable for producing signals in mw, the sensor also being designed to be connected to the CPU and the viewer.

Positioned beneath the lower radiation plate there is a tray made of a transparent plastic material suitable to protect the lower internal part of the instrument. The tray is removable, washable and disinfectable.

Reference numeral 8 refers to a section or segment of pipe realised in a synthetic material, with no memory, connectable to a pump element, in particular of a peristaltic type, the pump element being suitable for both the vehiculisation of a saline solution and for the recirculation of a cellular suspension, by means of a variable flow.

Numerals 7 and 11 refer to two sections or segments of pipe realised in a synthetic material, with no memory, connectable to electromagnetic clamps for managing liquid substances inside the disposable assembly 1 by means of the alternate opening and closure thereof.

Inside the instrument there is an optical-type haematocrit reader sensor present, the reader being controlled by a device 20, the sensor being suitable for reading the haematocrit and being connected to the central processing unit (CPU) and the instrument's viewer.

The central processing unit (CPU) with which the instrument is fitted is suitable for managing all the operative functions of which both the instrument and the disposable assembly are capable. The instrument is also fitted with a proximity reader (RFID device), the reader being connected to the central processing unit (CPU) and the instrument's viewer.

This proximity reader is provided to read and store the data which identifies the disposable assembly, to treat data inherent to each treatment executed and display the general instrument status. Connected to the aforesaid proximity reader is an antenna element which is encapsulated in a plastic/acrylic material and positioned on one side of the instrument, which is suitable for receiving signals coming from an RFID device 15 positioned in the disposable assembly 1.

There is a printer device integrated with the instrument, which is suitable for printing all the useful data to add to the patient's file or the work sheet to guarantee the certification and validation of the photoradiation treatment executed.

The instrument is also fitted with a cordless transmission system, of the Bluetooth or infrared or GSM type, designed to dialogue with one or more remote servers. Inside the photoradiation chamber there are two liquid presence sensors, positioned on the radiation plate in the zone below and in correspondence with the inlet and outlet of the photoradiation circuit pipes. The liquid presence sensors perform the important function of identifying possible zones where the biological fluid might leak out of the disposable assembly 1, the leakage being capable of producing considerable damage: both damage to the patient who, on the day of the leak, would lose the opportunity to carry out the therapy as a result of the loss of cells which could potentially have been infected during the biological fluid leak, and also damage caused to the operator in relation to the risk of infection with pathologies which could even be extremely serious (just think of HIV or HCV for example), to the time wasted reprogramming the treatment, the cost of the materials used and the time wasted cleaning and disinfecting the instrument.

The aforesaid instrument features, preferably on the front surface and housed in a suitable seat, two pressure sensors suitable for signalling eventual incongruous pressure values inside the disposable assembly circuit 1, the two pressure reading sensors being connected to the central processing unit (CPU) and the viewer.

The aforesaid instrument is also fitted with a sensor suitable for detecting the state of closure of the photoradiation chamber and is also fitted with a sensor suitable for detecting the state of closure of the peristaltic-type pump element cap.

The disposable assembly 1, according to the figure 1, is composed of a first pipeline 2 which, on one side, is connected to a first bag 3, external to the aforesaid disposable assembly, containing haematic cells collected from the patient with an appliance external to the present invention and, on the other side, it is connected to a recirculation bag 4.

A second pipeline 5 connects the recirculation bag 4 with a radiation bag 6. Positioned along the aforesaid second pipeline are sections or segments of dedicated pipe 7 and 8.

Situated upstream of the aforesaid section or segment of pipe 8 is the insertion between the second pipeline 5 and a third pipeline 9 which is connected to a second bag 10 external to the aforesaid disposable assembly and containing a saline solution; the section or segment of dedicated pipe 11 is positioned along the third pipeline 9.

Still with reference to the second pipeline 5, situated between the sections or segment of dedicated pipe 7 and 8 and upstream of the insertion of the third pipeline 9 with the second pipeline 5 is the connection between the latter and a first section or segment of dedicated pipe 12, containing a first pressure reading device 12a, which is inserted in the respective pressure sensor positioned on the instrument, and situated between the section or segment of dedicated pipe 8 and radiation bag 6 is the connection between the second pipeline 5 and a second section or segment of dedicated pipe 13, containing a second pressure reading device 13a, which is inserted in the respective pressure sensor also positioned on the instrument.

Positioned on the recirculation bag 4 is the radio frequency identification device 15 (RFID device) and a container for a photoactivating drug 16.

Still situated on the aforesaid recirculation bag are both a first inlet 17 with a "latex free"-type perforable guard suitable for the eventual collection of samples or the addition of extra drugs and substances, and also a second inlet 18 fitted with a protective cap, which is a device suitable for the transfer of cells for the reinfusion into the patient.

A fourth pipeline 19 joins the radiation bag 6 with the recirculation bag 4 and positioned along the fourth pipeline is a device 20 suitable for reading the haematocrit which must be inserted into at least one optical-type haematocrit reader sensor and positioned on the instrument.

An incubation bag 21 is connected to the recirculation bag 4 by means of a fifth pipeline 22 onto which are positioned two clamp elements 22a.

Situated on the aforesaid incubation bag, level with the recirculation bag 4, are both a first inlet 17 with a "latex free"-type perforable guard suitable for the eventual collection of samples or the addition of extra drugs and substances, and also a second inlet 18 fitted with a protective cap, which is a device suitable for the transfer of cells for the reinfusion into the patient.

After this predominantly structural description, there will now follow a description of the functioning of the invention in question with reference to the figures.

After switching on the instrument and fitting the disposable assembly 1 therein, it is important to connect, to the relative sensors present on the instrument, the portions of the disposable assembly 1 such as: the device 20 for reading the haematocrit (HCT), the devices for control in the pressure 12a and 13a, the section of segment of dedicated pipe 8 inside the peristaltic pump element, the sections or segments of dedicated pipe 7 and 11 to the electromagnetic clamps.

The RFID proximity reader will then read, in automatic mode, the data contained in the radio frequency identification (RFID) device 15, will enable the pliotoradiation procedure and will register all the identification data for the disposable assembly 1, and data concerning production, batch, expiry and complete product traceability.

There follows a first washing of the lines in the disposable assembly 1 using a saline solution, the lines being intended for the flow of the biological liquid to treat and the washing will also be effected automatically. The course followed will be as follows: first of all the saline solution will flow along the third pipeline 9, through the section or segment of dedicated pipe 11, it will reach the section or segment of dedicated pipe 8 and by means of the action of the peristaltic pump, will enter the radiation bag 6. On leaving the latter, it will flow through the fourth pipeline 19 and will arrive at the inlet in the recirculation bag 4. A known quantity of saline solution will be utilised in this washing operation.

The aforesaid saline solution flowing along the third pipeline 9 will be prevented from refluxing into the recirculation bag 4 by means of the automatic closure of the electromagnetic clamp on the section or segment of dedicated pipe 7. At the end of the washing of the disposable assembly 1, the instrument will stop and emit an acoustic warning signal while awaiting the entry of the data inherent to the cellular suspension to undergo photoradiation treatment which will be subsequently added to the recirculation bag 4, coming, by falling or by the force of gravity, through the first pipeline 2, from the first bag 3 containing the cellular suspension.

At this point, one proceeds by breaking the container 16 containing the photoactivating substance/drug which is consequently mixed with the cellular suspension contained in the recirculation bag 4, the mixing occurring in an optimal way by means of manual agitation of the recirculation bag.

Only after carrying out this operation can the instrument's start control be pressed and the UVA lamps will switch on, with the contemporaneous circulation of the physiological liquid inside the entire disposable assembly 1. During this circulation through the haematocrit reading device 20, the optical-type haematocrit reader sensor positioned on the instrument will proceed with the measurement of the HCT of the final cellular suspension, i.e. the value inherent to the HCT in the aforesaid suspension diluted with the saline solution which has been used to wash the pipelines of the disposable assembly 1 and the HCT value thus measured will be sent to the central processing unit (CPU); the CPU will utilise the haematocrit value measured, the value measured by the photometer and the values entered for the total volume of the suspension to treat, and will calculate and display the quantity of energy (expressed in Joules) to radiate inside the photoradiation chamber.

The circulation of the physiological liquid to radiate will follow the same course followed earlier by the saline solution during the washing of the lines in the disposable assembly 1 with the exception of the third pipeline 9 as the reflux of the physiological fluid is prevented by the automatic closure of the segment or section of dedicated pipe 11.

Over the course of the entire photoradiation process described above, the operator can keep a constant watch on the temperature inside the photoradiation chamber, the chamber temperature being constantly regulated by the cooling elements, and the correct functioning of the instrument by checking the data read by the sensors provided to monitor the value of the pressure of the liquid undergoing treatment, the existence of eventual leaks of the liquid, and the UVA radiation value, to give but a few examples; the monitoring of the correct functioning of the instrument 1 will be further facilitated by the insertion of specific sound alarms which will sound in the event of any anomaly.

When the photoradiation treatment ends, the UVA lamps switch off automatically and likewise a complete second wash of the disposable assembly 1 is executed automatically with saline solution in order to bring all the treated cells back into the recirculation bag 4 following the course described earlier for the first wash.

After effecting the aforesaid line washing, the instrument 1 stops, emitting contemporaneously, an acoustic signal, and the operator will be able to detach the recirculation bag 4 to be able, immediately after the photoradiation treatment ends, to reinfuse a percentage - on average, 50% - of the treated cells back into the patient again. The remaining part of the treated cellular suspension will then be brought into the incubation bag 21 using a fifth pipeline 22 connecting the recirculation bag 4 with the incubation bag. Once the photoradiation procedure has ended, all the data 5 inherent to both the disposable assembly 1 and the treatment will be registered in the CPU, which, at the operator's request can send it, in wireless transmission mode, to a remote unit. Naturally, all the data can be printed by means of the printing unit integrated into the instrument.

Finally, the assembly constituted of the central processing unit (CPU), the proximity reader, the radio frequency identification (RFID) device 15 situated on the recirculation bag and the wireless data transmission system will permit the entire photoradiation procedure to be monitored and also, at the end of each treatment, it permits the following to be obtained: the quantity of procedures effected and the quantity of disposable assemblies 1 utilised; the registration of all the data from each treatment executed necessary for the validation of the therapy; the monitoring of the conditions of the system, intending thereby the assembly comprising the instrument and the disposable assemblies utilised; the complete traceability of the product through the availability of all the identification data, and lastly, the guarantee of the product's authenticity and validity and the congruousness of the treatment.

The radio frequency identification (RFID) device 15 which is situated on each recirculation bag 4 will be authenticated by the proximity reader which authorises the effecting of the procedure and registers the effecting thereof thereby preventing both the use of falsified products and guaranteeing the circuit is used once only and preventing the falsification thereof after use.

As mentioned earlier, the treated cells can be reinfused into the patient either immediately after the completion of the treatment photoradiation or - even - a while after the end of the treatment by means of the second inlet 18; in the second eventuality, the presence of the gas exchanger device fitted with a 0.2 µ filter element 14 is exploited the filter element being positioned on the incubation bag 21, and the latter can be inserted into an incubator fitted with a thermostat and held there for the necessary time before the reinfusion into the patient of the photoradiated cells; positioned along the fifth pipeline 22 are two clamp elements 22a which means the fifth bag 21 is a closed circuit protected against any source of pollution. This is a procedure known as "transimmunisation" which is still in the research phase and necessitates incubation - at a temperature maintained constant with a thermostat - of the cells that have already undergone an extracorporeal photoimmunotherapy treatment lasting twelve hours, which means the incubation bag 21 will have to permit the passage of gas so as to guarantee the survival of the cells for their entire incubation period; by means of the use of the transimmunisation procedure it would seem possible to obtain greater therapeutic results than those obtained with the traditional type of extracorporeal photoimmunotherapy and with much more activity also with regards to pathologies that are little receptive to the aforesaid extracorporeal photoimmunotherapy.

The procedure is carried out during the functioning of the device described earlier. Over the course of the description, specific reference has been made to a photoactivating drug addable to the recirculation bag 4, the drug being contained in a container 16 which can be shattered or crushed but obviously, the procedure can function just as advantageously even if the photoactivating drug is added to the recirculation bag by means of the first inlet 17.

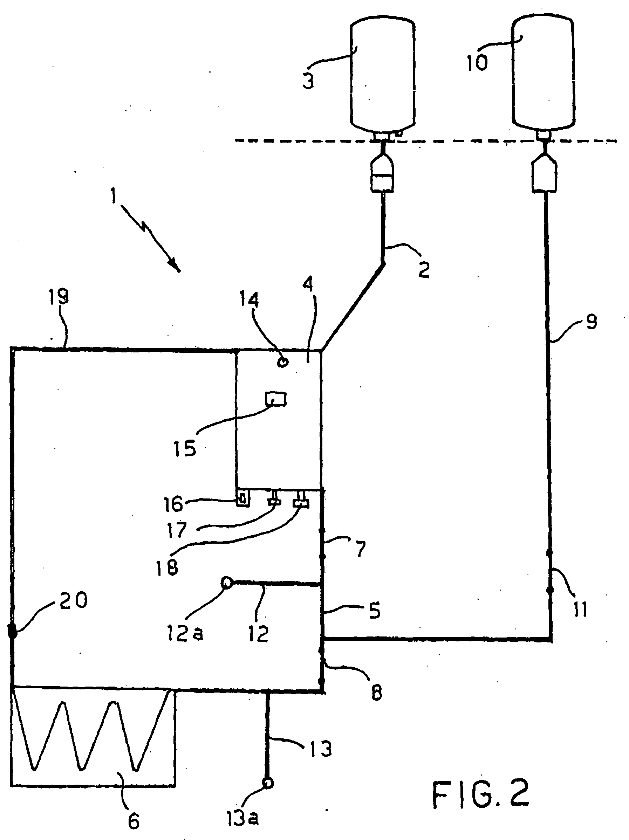

Again, over the course of the description, specific reference has been made to the presence of an incubation bag 21. Embodiments of the present disclosure can function just as advantageously even in the event that the disposable assembly 1 is lacking the aforesaid incubation bag 21, as long as the recirculation bag 4 is fitted with a 0.2 µ filter element 14, as shown in figure 2.

Embodiments of the present disclosure are arranged such that the collection of the buffy-coat is not envisaged, being realised instead with other devices external to the system in question in the present invention.

A first advantage of the present invention is constituted of the realisation of an appliance suitable for extracorporeal photoimmunotherapy which is simpler, less costly and more flexible with respect to the commonly known appliances.

A second advantage of the present invention consists in its rendering the extracorporeal photoimmunotherapy procedure safer, more standardised and faster.

A further advantage of the present invention is constituted of the possibility it offers to effect certification of the treatment executed as required by European standards on extracorporeal blood treatment.

A still further advantage is constituted of the possibility of effecting the transimmunisation therapy by maintaining a part of the cellular suspension (on average, 50% thereof) inside a container, i.e. the incubation bag 21, which constitutes a closed circuit with respect to the remaining disposable assembly; i.e. guaranteeing absolute pollution immunity to the percentage of the cellular suspension to reinfuse into the patient later on, after the photoimmunisation process has ended.

## Claims

1. A system for extracorporeal photoimmunotherapy of biological fluid, constituted of blood or fractions thereof, comprising an instrument and a disposable assembly (1), the instrument comprising at least one set of UVA lamps suitable for photoradiation, a radiation chamber for the insertion and disposition in the chamber of a radiation container (6) containing the fluid to undergo treatment, cooling elements for the radiation chamber and a pump element, the disposable assembly comprising a plurality of containers (4, 14) and pipelines (2, 5, 9, 22) for the containment and vehiculisation of the biological fluid, among which is a first pipeline (2), which on one side is for connection to an haematic cells container (3) for containing haematic cells, the assembly (1) also comprising the radiation container (6) and an accumulation container (4) for the intermediary storage of the blood or fractions thereof which is connected to the other side of said first pipeline (2); the instrument further compring the following elements:
(a) at least one couple of electromagnetic-type clamps, there being inserted, inside each of the clamps, sections of dedicated pipe (7, 11) present in the disposable assembly (1), the couple of electromagnetic clamps being suitable for managing liquids to undergo treatment by means of the alternate opening and closure of the containers (4, 14) and pipelines (2, 5, 9, 22) in the disposable assembly (1);
(b) at least one optical-type haematocrit reader sensor;
(c) at least two pressure sensors suitable to identify overpressure inside the plurality of containers (4,14)and pipelines (2, 5, 9,22) in the disposable assembly (1);
(d) at least two liquid presence sensors suitable to detect leaks of the liquid from the plurality of containers (4, 14) and pipelines (2, 5, 9,22) in the disposable assembly (1);
(e) an RFID proximity reader suitable for reading and storing product identification data for each disposable assembly (1),
(f) an arrangement for the transmission of data to a remote server; and the disposable assembly (1) further comprising
(g) a second pipeline (5) connecting said accumulation container (4) in the form of a recirculation container (4) to said radiation container (6), and along which is positioned one (7) of said sections of dedicated pipe;
(h) a third pipeline (9) connected to the second pipeline (5) and for connection to a saline solution container (10) for containing a saline solution, along which third pipeline one (11) of said sections of dedicated pipe is positioned; and
(i) an incubation container (21) connected to said recirculation container (4) by means of a further pipeline (22) onto which are positioned two clamp elements (22a).

2. A system according to claim 1, **characterised by** the fact that said instrument further comprises a central processing unit (CPU) suitable for managing all of the functions of the system.

3. A system according to claim 1 or 2, **characterised by** the fact that said instrument further comprises a serial port and/or integrated printer suitable to render possible the certification of each photoimmunotherapy treatment executed.

4. A system according to any one of the preceding claims, **characterised by** the fact that said instrument further comprises a protective cover element for electrical components of the instrument.

5. A system according to any preceding claims, **characterised by** the fact that, situated on the incubation container (21), level with the recirculation container (4), are both a first inlet (17) with a latex-free-type perforable guard suitable for the collection of samples or the addition of extra drugs and substances, and also a second inlet (18) fitted with a protective cap, which is a device suitable for the transfer of cells for the reinfusion into the patient.

6. A system according to any preceding claim, **characterised by** the fact that an antenna element is connected to the preliminary reader, the antenna element being encapsulated in a plastic/acrylic material and positioned on one side of the instrument, which is suitable for receiving signals coming from an RFID device (15) positioned in the disposable assembly (1).

7. A system according to any preceding claim, **characterised by** the fact that the two liquid presence sensors are situated, inside the radiation chamber, on a radiation plate in the zone below the inlet and outlet of radiation circuit pipes.

8. A system according to claim 1, **characterised by** comprising a photoactivating substance/drug container (16) for containing the photoactivating substance/drug and realised in a material which is not transparent to light and can easily be shattered or crushed inside the recirculation container(4).

9. A system according to any preceding claim, **characterised by** the fact that the sections of the pipes (7, 8, 11) are realised in a synthetic material with no memory.

10. A system according to any preceding claim, in which the arrangement for transmission of data to a remote server is GSM typology device.

11. A system according to any preceding claim, in which the two pressure sensors are positioned on the front side of the instrument.

## Patentansprüche

1. System zur extrakorporalen Photoimmuntherapie von biologischem Fluid, das aus Blut oder aus Teilen davon besteht, wobei das System ein Instrument und eine wegwerfbare Anordnung (1) umfasst, wobei das Instrument mindestens einen Satz von UVA-Lampen, die für eine Photobestrahlung geeignet sind, eine Bestrahlungskammer für die Einsetzung und Anordnung in der Kammer eines Bestrahlungsbehälters (6), der das Fluid enthält, das einer Behandlung unterzogen werden soll, Kühlelemente für die Bestrahlungskammer und ein Pumpelement umfasst, wobei die wegwerfbare Anordnung eine Vielzahl von Behältern (4, 14) und Rohrleitungen (2, 5, 9, 22) für die Einschließung und den Transport des biologischen Fluids umfasst, unter denen eine erste Rohrleitung (2) ist, die auf der einen Seite eine Verbindung mit einem Behälter (3) von Hämatinzellen aufweist, um Hämatinzellen aufzunehmen, wobei die Anordnung (1) auch den Bestrahlungsbehälter (6) und einen Akkumulationsbehälter (4) für die Zwischenlagerung des Blutes oder von Teilen davon, die mit der anderen Seite der ersten Rohrleitung (2) verbunden sind, umfasst; wobei das Instrument ferner die folgenden Elemente umfasst:
(a) mindestens ein Paar von Klemmen vom elektromagnetischen Typ, in die innerhalb jeder Klemme Abschnitte eines zugeordneten Rohres (7, 11), die in der wegwerfbaren Anordnung (1) vorhanden sind, eingesetzt werden, wobei das Paar von elektromagnetischen Klemmen geeignet ist, um Flüssigkeiten, die einer Behandlung unterzogen werden sollen, zu managen und zwar mittels eines abwechselnden Öffnens und Schließens der Behälter (4, 14) und der Rohrleitungen (2, 5, 9, 22) in der wegwerfbaren Anordnung (1);
(b) mindestens einen Hämatokrit-Ablesesensor vom optischen Typ;
(c) mindestens zwei Drucksensoren, die geeignet sind, um innerhalb der Vielzahl von Behältern (4, 14) und Rohrleitungen (2, 5, 9, 22) in der wegwerfbaren Anordnung (1) einen Überdruck zu identifizieren;
(d) mindestens zwei Flüssigkeitsnachweissensoren, die geeignet sind, um Flüssigkeitslecks aus der Vielzahl von Behältern (4, 14) und Rohrleitungen (2, 5, 9, 22) in der wegwerfbaren Anordnung (1) zu erkennen;
(e) einen berührungslosen RFID-Leser, der geeignet ist, um für jede wegwerfbare Anordnung (1) Produkt-Identifikationsdaten zu lesen und zu speichern;
(f) eine Anordnung für die Übertragung von Daten an einen Remote |Server|[AC1];
und wobei die wegwerfbare Anordnung (1) ferner umfasst:
(g) eine zweite Rohrleitung (5), die den Akkumulationsbehälter (4) in der Form eines Rezirkulationsbehälters (4) mit dem Bestrahlungsbehälter (6) verbindet, und wobei entlang dieser einer (7) der Abschnitte eines zugeordneten Rohres positioniert ist;
(h) eine dritte Rohrleitung (9), die mit der zweiten Rohrleitung (5) verbunden ist und der Verbindung mit einem Salzlösungsbehälter (10) dient, um eine Salzlösung aufzunehmen, und wobei entlang dieser dritten Rohrleitung einer (11) der Abschnitte eines zugeordneten Rohres positioniert ist; und
(i) einen Inkubationsbehälter (21), der mit dem Rezirkulationsbehälter (4) mittels einer weiteren Rohrleitung (22) verbunden ist, auf der zwei Klemmelemente (22a) positioniert sind.

2. System nach Anspruch 1, **gekennzeichnet durch** die Tatsache, dass das Instrument ferner eine zentrale Verarbeitungseinheit (CPU) umfasst, die in der Lage ist, alle Systemfunktionen zu managen.

3. System nach Anspruch 1 oder 2, **gekennzeichnet durch** die Tatsache, dass das Instrument ferner eine serielle Anschlussschnittstelle und/oder einen integrierten Drucker umfasst, die bzw. der geeignet ist, die Zertifizierung jeder Behandlung mit einer Photoimmuntherapie, die ausgeführt worden ist, möglich zu machen.

4. System nach einem der vorhergehenden Ansprüche, **gekennzeichnet durch** die Tatsache, dass das Instrument ferner zum Schutz für elektrische Komponenten des Instruments ein Abdeckungselement umfasst.

5. System nach einem der vorhergehenden Ansprüche, **gekennzeichnet durch** die Tatsache, dass sich auf dem Inkubationsbehälter (21) auf gleicher Höhe mit dem Rezirkulationsbehälter (4) sowohl ein erster Einlass (17) mit einem perforierbaren Schutz vom Typ eines latexfreien Schutzes, der für die Sammlung von Proben oder für die Zugabe von Extramedikamenten und Substanzen geeignet ist, als auch ein zweiter Einlass (18) befindet, der mit einer Schutzkappe ausgestattet ist, die eine Vorrichtung ist, die für die Übertragung von Zellen für die Reinfusion in den Patienten geeignet ist.

6. System nach einem vorhergehenden Anspruch, **gekennzeichnet durch** die Tatsache, dass ein Antennenelement mit der Vorablesevorrichtung verbunden ist, wobei das Antennenelement in einem Kunststoff-/Acrylmaterial eingeschlossen und auf einer Seite des Instruments positioniert ist, die geeignet ist, um Signale, die von einer RFID-Vorrichtung (15), die in der wegwerfbaren Anordnung (1) positioniert ist, zu empfangen.

7. System nach einem vorhergehenden Anspruch, **gekennzeichnet durch** die Tatsache, dass sich die zwei Flüssigkeitsnachweissensoren innerhalb der Bestrahlungskammer auf einer Bestrahlungsplatte in der Zone unterhalb des Einlasses und Auslasses von Rohren eines Bestrahlungskreislaufes befinden.

8. System nach Anspruch 1, **dadurch gekennzeichnet, dass** es einen photoaktiven Substanz-/Medikamentenbehälter (16) umfasst, um die photoaktive Substanz/das photoaktive Medikament aufzunehmen, und dass es in einem Material realisiert wird, das nicht lichtdurchlässig ist und das innerhalb des Rezirkulationsbehälters (4) nicht leicht zerspringen oder zerbersten kann.

9. System nach einem vorhergehenden Anspruch, **gekennzeichnet durch** die Tatsache, dass die Abschnitte der Rohre (7, 8, 11) in einem synthetischen Material ohne einen Speicherung realisiert werden.

10. System nach einem vorhergehenden Anspruch, wobei die Anordnung für eine Datenübertragung an einen Remote Server eine Vorrichtung von der GSM-Typologie ist.

11. System nach einem vorhergehenden Anspruch, wobei die zwei Drucksensoren auf der Vorderseite des Instruments positioniert sind.

## Revendications

1. Système de photo-immunothérapie extracorporelle d'un fluide biologique, constitué de sang ou de fractions de celui-ci, comprenant un instrument et un dispositif jetable (1), l'instrument comprenant au moins un ensemble de lampes UVA appropriées pour un photo-rayonnement, une chambre de rayonnement pour l'insertion et la mise en place, dans la chambre, d'un récipient de rayonnement (6) contenant le fluide qui doit subir le traitement, des éléments de refroidissement pour la chambre de rayonnement, et un élément de pompe, le dispositif jetable comprenant une pluralité de récipients (4, 14) et de canalisations (2, 5, 9, 22) pour le confinement et le transport du fluide biologique, incluant, parmi elles, une première canalisation (2) qui, d'un côté, est prévue pour un raccordement à un récipient de cellules hématiques (3) destiné à contenir des cellules hématiques, le dispositif (1) comprenant également le récipient de rayonnement (6) et un récipient d'accumulation (4) prévu pour le stockage intermédiaire du sang ou des fractions de celui-ci et qui est raccordé à l'autre côté de ladite première canalisation (2) ; l'instrument comprenant en outre les éléments suivants :
(a) au moins un couple d'éléments de serrage de type électromagnétique, dans lesquels sont insérés, à l'intérieur de chacun des éléments de serrage, des tronçons de conduite (7, 11) présents dans le dispositif jetable (1), le couple d'éléments de serrage électromagnétiques étant apte à gérer des liquides pour qu'ils subissent un traitement au moyen de l'ouverture et de la fermeture alternées des récipients (4, 14) et des canalisations (2, 5, 9, 22) dans le dispositif jetable (1) ;
(b) au moins un capteur lecteur d'hématocrite de type optique ;
(c) au moins deux capteurs de pression aptes à identifier une surpression à l'intérieur de la pluralité de récipients (4, 14) et de canalisations (2, 5, 9, 22) dans le dispositif jetable (1) ;
(d) au moins deux capteurs de présence de liquide aptes à détecter des fuites du liquide à partir de la pluralité de récipients (4, 14) et de canalisations (2, 5, 9, 22) dans le dispositif jetable (1) ;
(e) un lecteur de proximité RFID apte à lire et à mémoriser des données d'identification de produit pour chaque dispositif jetable (1) ;
(f) un agencement destiné à la transmission de données à un serveur à distance ;
et le dispositif jetable (1) comprenant en outre :
(g) une deuxième canalisation (5) raccordant ledit récipient d'accumulation (4), sous la forme d'un récipient de remise en circulation (4), audit récipient de rayonnement (6), et le long de laquelle est positionné l'un (7) desdits tronçons de conduite dédiée ;
(h) une troisième canalisation (9) raccordée à la deuxième canalisation (5) et prévue pour un raccordement à un récipient de solution saline (10) destiné à contenir une solution saline, l'un (11) desdits tronçons de conduite dédiée étant positionné le long de cette troisième canalisation ; et
(i) un récipient d'incubation (21) raccordé audit récipient de remise en circulation (4) au moyen d'une canalisation supplémentaire (22) sur laquelle sont positionnés deux éléments de serrage (22a).

2. Système selon la revendication 1, **caractérisé par le fait que** ledit instrument comprend en outre une unité centrale de traitement (CPU) apte à gérer la totalité des fonctions du système.

3. Système selon la revendication 1 ou 2, **caractérisé par le fait que** ledit instrument comprend en outre un port série et/ou une imprimante intégrée apte(s) à rendre possible la certification de chaque traitement de photo-immunothérapie effectué.

4. Système selon l'une quelconque des revendications précédentes, **caractérisé par le fait que** ledit instrument comprend en outre un élément de couverture de protection pour les composants électriques de l'instrument.

5. Système selon une quelconque revendication précédente, **caractérisé par le fait que** sont prévues, en étant situées sur le récipient d'incubation (21), au niveau du récipient de remise en circulation (4), à la fois une première entrée (17), avec une protection perforable du type sans latex appropriée pour le recueil d'échantillons ou l'addition de médicaments et de substances supplémentaires, et, également, une deuxième entrée (18) pourvue d'un capuchon de protection, formant un dispositif approprié pour le transfert de cellules en vue d'une nouvelle perfusion chez le patient.

6. Système selon une quelconque revendication précédente, **caractérisé par le fait qu'**un élément d'antenne est relié au lecteur préliminaire, l'élément d'antenne étant encapsulé dans un matériau plastique/acrylique et positionné sur un côté de l'instrument, et étant approprié pour recevoir des signaux provenant d'un dispositif RFID (15) positionné dans le dispositif jetable (1).

7. Système selon une quelconque revendication précédente, **caractérisé par le fait que** les deux capteurs de présence de liquide sont situés, à l'intérieur de la chambre de rayonnement, sur une plaque de rayonnement dans la zone située au-dessous de l'entrée et de la sortie des conduits du circuit de rayonnement.

8. Système selon la revendication 1, **caractérisé en ce qu'**il comprend un récipient (16) de substance ou de médicament de photo-activation, destiné à contenir la substance ou le médicament de photo-activation et réalisé en un matériau qui n'est pas transparent à la lumière et qui peut être facilement brisé ou broyé à l'intérieur du récipient de remise en circulation (4).

9. Système selon une quelconque revendication précédente, **caractérisé par le fait que** les tronçons des conduites (7, 8, 11) sont réalisés en un matériau synthétique sans mémoire.

10. Système selon une quelconque revendication précédente, dans lequel l'agencement destiné à la transmission de données à un serveur à distance est un dispositif selon la technologie GSM.

11. Système selon une quelconque revendication précédente, dans lequel les deux capteurs de pression sont positionnés sur la face avant de l'instrument.
